Europäisches Patentamt

European Patent Office

Office européen des brevets

(11) Publication number: **0 648 487 A1**

# EUROPEAN PATENT APPLICATION

(21) Application number: **94115764.6**

(22) Date of filing: **06.10.94**

(51) Int. Cl.⁶: **A61K 9/36**, A61K 9/42,
A61K 47/12, A61K 47/14,
A61K 47/38

(30) Priority: **08.10.93 JP 252989/93**

(43) Date of publication of application:
**19.04.95 Bulletin 95/16**

(84) Designated Contracting States:
**CH DE FR GB LI**

(71) Applicant: **Shin-Etsu Chemical Co., Ltd.**
**6-1, Ohtemachi 2-chome**
**Chiyoda-ku**
**Tokyo 100 (JP)**

(72) Inventor: **Maruyama, Naosuke, Synthetic**
**Techn. Lab.**
**Shin-Etsu Chem. Co. Ltd.**
**28-1 Oaza Nishi-Fukushima**
**Kubiki-mura**
**Nakakubiki-gun**
**Niigata 94201 (JP)**
Inventor: **Kokubo, Hiroyasu, Synthetic Techn.**
**Lab.**
**Shin-Etsu Chem. Co. Ltd.**
**28-1 Oaza Nishi-Fukushima**
**Kubiki-mura**
**Nakakubiki-gun**
**Niigata 94201 (JP)**

(74) Representative: **Tiedtke, Harro, Dipl.-Ing.**
**Patentanwaltsbüro**
**Tiedtke-Bühling-Kinne & Partner**
**Bavariaring 4**
**D-80336 München (DE)**

(54) **Dispersion of enteric coating agent.**

(57) A dispersion of an enteric coating agent which can hold its uniform and stable dispersed condition over a long period of time without causing any aggregation even if it is exposed to any temperature change and which has high safety as a component for preparing pharmaceutical preparations is herein disclosed. The dispersion comprises an enteric coating base, a plasticizer and an anionic surfactant and is characterized in that the enteric coating base having an average particle size of not more than 10 μm is dispersed in water in a concentration ranging from 5 to 15% by weight on the basis of the total weight of the dispersion and that the ratio, the enteric coating agent: the plasticizer: the anionic surfactant is 100 parts by weight: 15 to 40 parts by weight: 0.1 to 10 parts by weight. The enteric coating base is preferably either hydroxypropyl methyl cellulose phthalate or hydroxypropyl methyl cellulose acetate succinate and the plasticizer is preferably either triethyl citrate or triacetin.

The present invention relates to a dispersion of a coating base which is applied onto the surface of a solid enteric pharmaceutical preparation.

The solid enteric pharmaceutical preparation is provided with an enteric coating film for the purposes of protecting drugs showing low resistance to acids from the attack of the acid in the stomach, of protecting the gastric mucous membrane from the attack of drugs which may stimulate and damage the wall of the stomach and is dissolved after the arrival at the intestines in which the pharmaceutical preparation shows its pharmacological action.

There have been used acrylic polymers and cellulosic polymers as enteric coating bases for such dispersion. The acrylic polymer may be, for instance, an emulsion polymer of methacrylic acid and ethyl acrylate. Examples of cellulosic polymers are cellulose acetate phthalate (CAP), hydroxypropyl methyl cellulose phthalate (HPMCP), hydroxypropyl methyl cellulose acetate succinate (HPMCAS) and carboxymethyl ethyl cellulose (CMEC). These coating bases are used in the coating treatment of drugs in the form of a solution in an organic solvent or an aqueous latex or an aqueous dispersion. Recently, the use of organic solvents has been regulated due to the problem of environmental pollution and correspondingly, coating methods which make use of an aqueous system such as a latex or an aqueous dispersion have widely been adopted.

There have already been proposed a variety of coating techniques which make use of aqueous systems, such as those described below. Japanese Patent Application Publication No. 60-43334 discloses an emulsion of an acrylic coating base. The acrylic coating base is an emulsion polymer of methacrylic acid and ethyl acrylate, has a particle size of not more than 1 $\mu$m and forms a stable emulsion. However, a polymerization initiator, a chain-transfer agent, unreacted monomers or the like are included in the enteric coating film as impurities and thus the coating film serving as a component of a medicine may suffer from a problem of safety.

To disperse a cellulosic coating base in water, there have been known a method comprising adding salts along with the coating base or neutralizing the carboxyl groups of the coating base and a method comprising dispersing an enteric polymer in water in the form of fine particles.

With regard to the former method, Japanese Patent Application Publication No. 61-56221 discloses a method which comprises the steps of emulsifying CAP, then adding a phosphoric acid salt as an anti-flocculating agent and spray-drying the resulting emulsion. The emulsification of CAP is performed according to the method disclosed in U.S. Patent No. 4,177,177. The spray-drying process permits the production of enteric polymer powder capable of being easily dispersed in water. In addition, Japanese Patent Provisional Publication No. 56-30913 discloses a method comprising neutralizing an aqueous solution of CAP or HPMCP with ammonia. Moreover, Japanese Patent Provisional Publication No. 58-135807 discloses a method which comprises the steps of neutralizing a cellulosic coating base with an alkali and simultaneously dissolving it therein and then adding a carboxylic acid. In cases of the dispersion neutralized with ammonia and the dispersion obtained by neutralizing with an alkali and then adding a carboxylic acid, ammonium salts and alkali salts of the carboxylic acid may often remain in the resulting enteric coating films. These salts are highly hygroscopic and thus may deteriorate the quality of the medicines to be coated.

With regard to the latter method, Japanese Patent Application Publication No. 56-12614 discloses a method in which fine particles of an enteric polymer are dispersed in water. In this method, a particulate enteric cellulose derivative having an average particle size of not more than 100 $\mu$m is dispersed in water containing a gelatinizing agent (plasticizer) having a boiling point of not higher than 100°C. Japanese Patent Application Publication numbers 57-53329 and 58-55125 disclose, in connection with the foregoing method, triacetin or triethyl citrate as a gelatinizing agent used in such method. The contamination of the resulting enteric coating film with, for instance, polymerization initiators and ammonium salts would be inhibited if a gelatinizing agent is dispersed in water and then a coating base is dispersed therein. In such dispersions, however, the coating base molecules undergo aggregation due to any temperature rise in the outer world and are in turn precipitated. More specifically, the coating base is quite sensitive to temperature. If the coating base undergoes precipitation, the resulting precipitates cause clogging of a spray tube and the nozzle of a spray gun during spray-coating a pharmaceutical component to be coated and become a cause of various troubles. To prevent the occurrence of any aggregation of the coating base particles, the temperature of the dispersion must always be maintained at a level of not more than 20 °C till the dispersion is put into practical use in the coating treatment and the gelatinizing agent must likewise be used in a relatively large amount. For this reason, it takes much labor to prepare such dispersion.

Moreover, Japanese Patent Application Publication No. 60-502207 discloses a dispersion of a coating agent which is prepared by dispersing polyvinyl alcohol phthalate (PVAP) in water through the addition of ammonia. The enteric pharmaceutical preparations coated with such a dispersion of a coating agent are quite hygroscopic and liable to be easily deteriorated.

The present invention has been completed for solving the foregoing problems associated with the conventional techniques and, therefore, it is an object of the present invention to provide a dispersion of an enteric coating agent which can hold its uniform and stable dispersed condition over a long period of time without causing any aggregation even if it is exposed to any temperature change and which has high safety as a component for preparing pharmaceutical preparations.

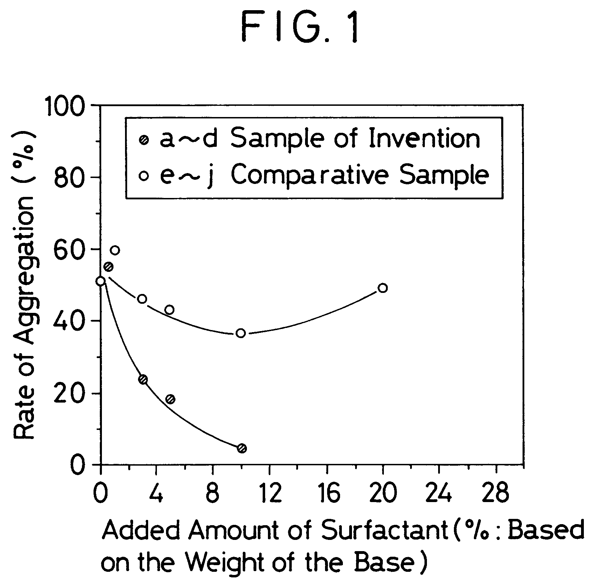

Fig. 1 shows the difference in the rate of aggregation observed when an anionic surfactant and nonionic surfactant are used as surfactants.

The foregoing object of the present invention can effectively be accomplished by providing a dispersion of an enteric coating agent which comprises an enteric coating base, a plasticizer and an anionic surfactant and which is characterized in that the enteric coating base having an average particle size of not more than 10 $\mu$m is dispersed in water in a concentration ranging from 5 to 15% by weight on the basis of the total weight of the dispersion and that the ratio, the enteric coating agent: the plasticizer: the anionic surfactant is 100 parts by weight: 15 to 40 parts by weight: 0.1 to 10 parts by weight.

Examples of the enteric coating bases used in the dispersion of the present invention are hydroxypropyl methyl cellulose phthalate (HPMCP) and hydroxypropyl methyl cellulose acetate succinate (HPMCAS). These HPMCP and HPMCAS may be used alone or in combination. The particle size thereof is preferably not more than 10 $\mu$m. This is because if it exceeds 10 $\mu$m, it is difficult to form a dense film. The particle size is preferably determined by the Fraunhofer Diffraction Method while using laser rays.

The coating base is used in combination with the plasticizer. Examples of such plasticizers are triethyl citrate and triacetin. These plasticizers may likewise be used alone or in combination. Triethyl citrate is preferably used and the use thereof ensures highly stable dispersibility of the coating base in an aqueous medium.

Examples of anionic surfactants used in the dispersion along with the plasticizer include sodium alkyl sulfates such as sodium lauryl sulfate and sodium dioctyl sulfosuccinate. Examples of such anionic surfactants further include sodium and potassium salts of fatty acids such as sodium oleate and potassium sorbate. These surfactants are anionic surfactants which can be used as components for preparing pharmaceutical preparations with high safety. These anionic surfactants may be used alone or in any combination.

The dispersion of the enteric coating agent according to the present invention may, if necessary, comprise an anti-tack agent and/or a coloring agent. The dispersion may further comprise, for instance, a corrigent for improving the taste upon oral administration and for concealing an unpleasant smell of the drug to be coated and small amounts of other plasticizers for improving the film-forming ability of the dispersion. In addition, the dispersion may likewise comprise a water-soluble or water-dispersible high molecular weight substance.

The concentration of the coating base ranges from 5 to 15% by weight and preferably 7 to 10% by weight on the basis of the total weight of the dispersion. This is because if the concentration of the base is less than 5% by weight, the coating treatment requires a long time and this results in insufficient productivity, while if it exceeds 15% by weight, the coating base easily undergoes aggregation when the temperature thereof rises and the resulting dispersion becomes unstable.

The amount of the plasticizer such as triethyl citrate or triacetin suitably ranges from 15 to 40 parts by weight per 100 parts by weight of the coating base. If HPMCP is used as the coating base, the amount of the plasticizer used is preferably in the range of from 25 to 40 parts by weight. If the amount thereof is less than 25 parts by weight, the resulting dispersion does not form a satisfactory enteric coating film. On the other hand, if it exceeds 40 parts by weight, the rate of the plasticizer is extremely high, this promotes the aggregation of the coating base molecules and thus a stable dispersion cannot be obtained. If HPMCAS is used as the coating base, the amount of the plasticizer to be added preferably ranges from 15 to 30 parts by weight. HPMCAS can easily be formed into a film as compared with HPMCP. For this reason, HPMCAS can form a satisfactory film even when the amount of the plasticizer is less than 25 parts by weight, but if the amount of the plasticizer is less than 15 parts by weight, it is difficult to form a satisfactory enteric coating film. On the other hand, if it exceeds 30 parts by weight, the rate of the plasticizer is too high, this promotes the aggregation of the coating base molecules and it is thus difficult to form a stable coating base solution.

The amount of the anionic surfactant to be added to the dispersion is in the range of from 0.1 to 10 parts by weight and preferably 3 to 5 parts by weight per 100 parts by weight of the coating base. If the amount of the surfactant is less than 0.1 part by weight, the coating base molecules easily undergo aggregation even if the temperature is only slightly raised and any stable dispersion cannot be provided. On the other hand, if it exceeds 10 parts by weight, a stable dispersion can be obtained and the coating base molecules never undergo any aggregation even when the dispersion is exposed to a temperature change, but the resulting film has low resistance to acids, is easily damaged by the action of the acid in the stomach and dissolved in the gastric juice prior to its arrival at the intestines. The problem of premature dissolution may also arise because the anionic surfactant per se is highly soluble in water.

The dispersion of the enteric coating agent according to the present invention is suitably prepared in the following manner. First, the surfactant is dissolved in a desired amount of water, then the plasticizer is added to the aqueous solution and the coating base powder is added to the solution, with stirring, to a predetermined concentration. In this respect, it is also possible to prepare, in advance, an aqueous dispersion of the coating base having a high concentration and to then add the aqueous dispersion to the foregoing aqueous solution.

Such a dispersion of the enteric coating agent is used for applying an enteric coating film to solid drugs such as tablets, granules or capsules.

The coating treatment for forming an enteric coating film on the surface of a solid drug may be carried out according to the conventional methods which make use of, for instance, a pan coating device, a drum type coating device, a fluidized bed coating device or a stirred fluidized bed coating device. The spray device for spray-coating the surface of pharmaceutically effective components with the dispersion may be an air-spray type or airless-spray type one. In this respect, if solid components such as coloring agents are liable to cause sedimentation, the spray-coating is preferably carried out while stirring the dispersion. After completion of the spray-coating, the resulting drug carrying an enteric coating film thus formed may be subjected to appropriate necessary treatments such as drying, heating, polishing, application of a sugar coating and other coating treatments with other coating bases.

When an enteric coating film is formed on the surface of a solid drug, another coating base, for instance, a coating base soluble in the gastric juice such as hydroxypropyl methyl cellulose can be applied onto the drug to give a film soluble in the gastric juice prior to the application of the enteric coating film, for the purpose of facilitating the formation of the enteric coating film. This leads to the formation of an enteric pharmaceutical preparation highly resistant to the gastric juice.

In the dispersion of the present invention, the coating base having an average particle size of not more than 10 $\mu$m is dispersed in water together with the plasticizer due to the action of the anionic surfactant. The surfactant serves to improve the wettability of the surface of the coating base with water and the compatibility of the particles to water, to further maintain the particle size of the base particles as small as possible and to impart electrical repulsion force to the particles to thus reduce the velocity of collision therebetween and to prevent aggregation thereof, through adsorption on the particles. The particle size of the coating base particles is very small on the order of not more than 10 $\mu$m and accordingly, a quite dense film can easily be formed on solid enteric preparations.

In the dispersion of the present invention, the surfactant is adsorbed on the surface of the coating base particles to thus improve the wettability thereof with water and permits stable dispersion of the particles while maintaining a small particle size thereof. For this reason, the amount of the plasticizer can be reduced as compared with the amount thereof used in the conventional methods and the dispersion can be prepared at room temperature. The resulting dispersion of the coating agent hardly causes aggregation irrespective of any temperature change and the coating base particles are uniformly and stably dispersed in the medium over a long period of time. The production of the dispersion does not require any cooling treatment for preventing the reduction of the viscosity thereof and the enteric coating film can likewise be formed at room temperature. Moreover, the resulting enteric coating film would not be contaminated with any substance such as polymerization initiators and unreacted monomers which become a cause of a problem of safety and thus the resulting pharmaceutical preparations have high safety.

The present invention will hereunder be described with reference to the following working Examples.

Example 1

A dispersion of an enteric coating agent having the composition as listed in the following Table 1 was prepared using HPMCP (HP-55UF available from Shin-Etsu Chemical Co., Ltd.) as a coating base.

Prismatic granules (1.5 Kg) of pancreatin having a diameter of 0.8 mm were sprayed with the dispersion of the enteric coating agent thus prepared. The spraying treatment was performed using a coating device, Flow Coater FLO-1, and an ATF gun having a diameter of 1.8 mm which are both available from Freund Sangyo Co., Ltd. The spraying-air pressure was set at 2.0 Kg/cm$^2$ and the spraying velocity was adjusted to 60 g/min. After the spraying treatment, an air flow of 80 °C was fed to the prismatic granules of pancreatin having a temperature of 33 to 34 °C at a flow rate of 2.7 m$^3$/min to evaporate moisture from the surface thereof. The temperature of the exhaust gas was found to be 35 to 36 °C. Thus, there were prepared five kinds of enteric granules each having a rate of the film ranging from 12% to 20% by weight.

The amount of pancreatin dissolved and released into the gastric juice as the results of destruction of the enteric coating film of the enteric granules thus prepared was determined by the basket method according to Japanese Pharmacopoeia-12 using a dissolution tester. More specifically, a first solution (artificial gastric juice) having a pH of 1.2 was rotated at a velocity of 100 rpm while maintaining the solution at a temperature of 37 °C, the enteric granule was immersed in the solution for 2 hours to thus determine the amount of the protein released from the granule. The results thus obtained are summarized in the following Table 2.

Examples 2 and 3 and Comparative Example 1

Dispersions of enteric coating agents each having the corresponding composition listed in Table 1 were prepared in the same manner used in Example 1, enteric granules were likewise prepared and the amounts of protein released from these granules were determined according to the same procedures used in Example 1. The results thus observed are listed in Table 2.

The data listed in Table 2 clearly indicate that the resulting enteric coating film became hardly soluble in the gastric juice through the addition of an anionic surfactant and that the resistance to acids of the film was substantially improved.

Table 1

| (Unit: part by weight) | | | | |
|---|---|---|---|---|
| | Ex. 1 | Ex. 2 | Ex. 3 | Comp. Ex.1 |
| HPMCP | 10.0 | 10.0 | 10.0 | 10.0 |
| Triethyl Citrate | 3.5 | 3.5 | 3.5 | 3.5 |
| Sodium Lauryl Sulfate | 0.1 | 0.3 | 1.0 | 0 |
| Purified Water | 86.4 | 86.2 | 85.5 | 86.5 |

Table 2

| (Unit: % by weight) | | | | |
|---|---|---|---|---|
| Amount of Film Applied | Ex. 1 | Ex. 2 | Ex. 3 | Comp. Ex.1 |
| 12% | 14.8 | 11.4 | 26.7 | 42.9 |
| 14% | 2.9 | 2.8 | 12.7 | 23.6 |
| 16% | 1.2 | 0.8 | 2.7 | 6.8 |
| 18% | 0.9 | 0.6 | 1.3 | 1.4 |
| 20% | 0.7 | 0.4 | 1.0 | 0.2 |

⟨Aggregation-Inhibitory Effect of Anionic Surfactant Which is Compared with That of Nonionic Surfactant⟩

There was examined the difference in the rate of aggregation between an anionic and nonionic surfactants.

Dispersions of enteric coating agents (Samples a to d) according to the present invention were prepared at a temperature of 15 °C so that each sample had the corresponding composition listed in the following Table 3. HPMCAS (Shin-Etsu AQOAT AS-MF available from Shin-Etsu Chemical Co., Ltd.) was used as a coating base and sodium lauryl sulfate was used as the anionic surfactant. In addition, Dispersions of enteric coating agents (Comparative Samples e to j) were likewise prepared in the same manner used above so that each sample had the corresponding composition listed in Table 3. HPMC similar to HPMCAS used in Example 1 was used as a coating base and the surfactant used was a nonionic surfactant (Polysolvate 80 available from Nikko Chemicals Co., Ltd.).

### Table 3 (Unit: part by weight)

| | Sample no. | | | |
|---|---|---|---|---|
| | a | b | c | d |
| HPMCP | 10 | 10 | 10 | 10 |
| Triethyl Citrate | 3.5 | 3.5 | 3.5 | 3.5 |
| Anionic Surfactant | 0.01 | 0.3 | 0.5 | 1.0 |
| Nonionic Surfactant | | | | |
| Total Amount(inclusive of Purified Water) | 100 | 100 | 100 | 100 |

### Table 3 (continued)

| | Comparative Sample no. | | | | | |
|---|---|---|---|---|---|---|
| | e | f | g | h | i | j |
| HPMCP | 10 | 10 | 10 | 10 | 10 | 10 |
| Triethyl Citrate | 3.5 | 3.5 | 3.5 | 3.5 | 3.5 | 3.5 |
| Anionic Surfactant | | | | | | |
| Nonionic Surfactant | 0 | 0.2 | 0.3 | 0.5 | 1.0 | 2.0 |
| Total Amount(inclusive of Purified Water) | 100 | 100 | 100 | 100 | 100 | 100 |

Sample a (800 ml) was introduced into a dissolution tester and rotated at 40 °C and a rate of 200 rpm for one hour with stirring. Then Sample was passed through a 60-mesh sieve, followed by drying aggregates remaining on the sieve, determination of the weight of the aggregates to thus inspect the sample for the rate of aggregation. The results thus obtained are plotted on Fig. 1.

Samples b to j were likewise inspected for the rates of aggregation according to the same procedures used above. The results are also plotted on Fig. 1.

In Fig. 1, the amount of the surfactant added is plotted as abscissa and the rate of aggregation observed at 40 °C is plotted as ordinate. In this respect, the amount of the surfactant added is expressed in terms of the weight thereof (part by weight) per 100 parts by weight of the coating base.

It was found that HPMCP was highly stably dispersed in an aqueous medium even at 40 °C through the addition of an anionic surfactant such as sodium lauryl sulfate. On the other hand, it was also found that such an effect was not observed at all through the addition of a nonionic surfactant.

A dispersion of an enteric coating agent which can hold its uniform and stable dispersed condition over a long period of time without causing any aggregation even if it is exposed to any temperature change and which has high safety as a component for preparing pharmaceutical preparations is herein disclosed. The dispersion comprises an enteric coating base, a plasticizer and an anionic surfactant and is characterized in that the enteric coating base having an average particle size of not more than 10 $\mu$m is dispersed in water in a concentration ranging from 5 to 15% by weight on the basis of the total weight of the dispersion and that the ratio, the enteric coating agent: the plasticizer: the anionic surfactant is 100 parts by weight: 15 to 40 parts by weight: 0.1 to 10 parts by weight. The enteric coating base is preferably either hydroxypropyl methyl cellulose phthalate or hydroxypropyl methyl cellulose acetate succinate and the plasticizer is preferably either triethyl citrate or triacetin.

**Claims**

1. A dispersion of an enteric coating agent which comprises an enteric coating base, a plasticizer and an anionic surfactant, wherein the enteric coating base having an average particle size of not more than 10 $\mu$m is dispersed in water in a concentration ranging from 5 to 15% by weight on the basis of the total weight of the dispersion and the ratio, the enteric coating agent: the plasticizer: the anionic surfactant is 100 parts by weight: 15 to 40 parts by weight: 0.1 to 10 parts by weight.

2. The dispersion of claim 1 wherein the enteric coating base is at least one member selected from the group consisting of hydroxypropyl methyl cellulose phthalate and hydroxypropyl methyl cellulose acetate succinate.

3. The dispersion of claim 1 wherein the plasticizer is at least one member selected from the group consisting of triethyl citrate and triacetin.

4. The dispersion of claim 1 wherein the anionic surfactant is at least one member selected from the group consisting of sodium alkyl sulfate, sodium salts of fatty acids and potassium salts of fatty acids.

# FIG. 1

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,Y | US-A-4 543 370 (PORTER ET AL.) 24 September 1985 <br> * see column 1 line 50 -column 2 line 45; column 9 lines 5-15, example 1 * <br> --- | 1-4 | A61K9/36 <br> A61K9/42 <br> A61K47/12 <br> A61K47/14 <br> A61K47/38 |
| X <br> Y | US-A-5 206 030 (WHEATLEY) 27 April 1993 <br> * see the whole document, especially claims 1,4 and 6 * <br> --- | 1,3,4 <br> 1-4 | |
| Y | PATENT ABSTRACTS OF JAPAN <br> vol. 9, no. 17 (C-262) (1740) 24 January 1985 <br> & JP-A-59 167 521 (SHINETSU KAGAKU KOGYO) 21 September 1984 <br> * abstract * <br> --- | 1-4 | |
| A | PATENT ABSTRACTS OF JAPAN <br> vol. 7, no. 219 (C-188) (1364) 29 September 1983 <br> & JP-A-58 118 511 (FUROINTO SANGYO) 14 July 1983 <br> * abstract * <br> --- | 1-3 | TECHNICAL FIELDS SEARCHED (Int.Cl.6) <br><br> A61K |
| A | PHARMACEUTICAL TECHNOLOGY, <br> vol.11, no.2, February 1987 <br> pages 26 - 32 <br> F. GUMOWSKI ET AL. 'The use of a new redispersible aqueous enteric coating material' <br> * see pages 26 and 28 * <br> ----- | 1-3 | |

The present search report has been drawn up for all claims

| Place of search | Date of completion of the search | Examiner |
|---|---|---|
| MUNICH | 8 February 1995 | Isert, B |

EPO FORM 1503 03.82 (P04C01)